# EUROPÄISCHE PATENTANMELDUNG

(11) **EP 1 676 561 A2**
(43) Veröffentlichungstag der Anmeldung: **05.07.2006**
(21) Anmeldenummer: 05023206.5
(22) Anmeldetag: 25.10.2005
(51) Int. Cl.: A61K 8/18

(54) **Ein biokatalytisches Mehrphasensystem enthaltende Zubereitungen sowie deren Anwendung**

(30) Priorität: 26.11.2004 DE 102004057150
(71) Anmelder: Merz Pharma GmbH & Co.KGaA, 60318 Frankfurt (DE)
(72) Erfinder: Beutler, Rolf D. Dr., 64739 Höchst / Hummetroth (DE); Schmidt, Karl Heinz Prof. Dr. Dr., 72810 Gomaringen (DE); Funke, Bettina, 60316 Frankfurt/Main (DE)
(74) Vertreter: Müller, Claudia

(57) **Zusammenfassung**

Die vorliegende Erfindung betrifft topisch applizierbare Zubereitungen mit einer Kombination aus einem biokatalytischen Mehrphasensystem und einem Kapillar- Wirksystem. Dabei werden insbesondere Transferproteine und lipophile /amphiphile Substanzen einerseits mit einer kapillarverbessernden Komponente andererseits eingesetzt. Dies bedingt einen gesteigerten Transfer lipophiler /amphiphiler Substanzen zum gewünschten Wirkort zusammen mit einer Aktivierung der Durchblutung am Wirkort und führt insgesamt zu einer unerwartet gesteigerten Effizienz sowohl transportierter Wirkstoffe als auch im System bereits auf andere Weise vorhandener Wirkstoffe. Die Zubereitungen können insbesondere kosmetisch und auch dermatologisch- pharmazeutisch eingesetzt werden.

## Beschreibung

Die vorliegende Erfindung betrifft topisch applizierbare Zubereitungen mit einer Kombination aus einem biokatalytischen Mehrphasensystem und einem Kapillar- Wirksystem. Dabei werden insbesondere Transferproteine und lipophile /amphiphile Substanzen einerseits mit einer kapillarverbessernden Komponente andererseits eingesetzt. Dies bedingt einen gesteigerten Transfer lipophiler /amphiphiler Substanzen zum gewünschten Wirkort zusammen mit einer Aktivierung der Durchblutung am Wirkort und führt insgesamt zu einer unerwartet gesteigerten Effizienz sowohl transportierter Wirkstoffe als auch im System bereits auf andere Weise vorhandener Wirkstoffe. Die Zubereitungen können kosmetisch und auch dermatologisch- pharmazeutisch eingesetzt werden.

### Hintergrund der Erfindung

Topisch anwendbare Zubereitungen zielen im Allgemeinen darauf ab, die Haut, -Anhangsgebilde, oder Schleimhäute mit einem oder mehreren Wirkstoffen zu versorgen. Im kosmetischen / dermatologisch- pharmazeutischen Bereich werden hierzu meist geeignete Emulsionen (wie z. B. O/W, W/O- Cremes, Lotionen), Fluids oder Gele eingesetzt. Sie enthalten den oder die Wirkstoffe zugesetzt in der Annahme, dass der Stoff auch das angestrebte Ziel (Wirkort) sicher erreicht und darüber hinaus auch über eine ausreichende Wirkung verfügt. Insbesondere bei funktionsgestörter oder alternder Haut liegt zumeist eine Minderdurchblutung, eine verminderte Energetisierung und / oder eine mangelnde biokatalytische Aktivität vor, welche dann zu Deformationen an der Oberflächenstruktur der Haut wie z. B. Falten, Orangenhaut etc. und umgekehrt auch zu erschwertem Wirkstofftransport führen. Zur Vermeidung derartiger Phänomene werden Wirkstoffe in unterschiedlicher galenischer Zubereitung eingesetzt. So beschreibt die DE 100 61 419 die Verwendung von Proteinen als Wirkstoff zur Restrukturierung von Haaren. In der CH B 686 554 werden kosmetische Zubereitungen beschrieben aus Proteinen und Wasser / Glycerin. Die WO 00/64472 beschreibt Zusammensetzungen zur Behandlung dermatologischer Störungen, enthaltend Fruchtextrakte wie Granatapfelextrakt, um freie Radikale zu neutralisieren, sowie Feuchtigkeitsmittel wie Hydroxypolycarboxylsäuren, Dimethicone, Glycerin , Natriumchlorid oder Weizenprotein.
Es wird also davon ausgegangen, dass der Wirkstoff so gut als möglich in die Haut penetriert und zum Wirkort gelangt, ohne an Effizienz einzubüßen. Hierzu können auch Penetrationsförderer wie Alkohole und analoge Substanzen zugesetzt werden. Ein weiteres Mittel zum Wirkstofftransport besteht im Einsatz von Liposomen. Dabei ist jedoch auf deren Konzentration und Stabilität zu achten. Günstigstenfalls kann mit derartigen Methoden ein passives Depot entstehen, da auch bei Liposomen das Problem der Überwindung der Energiebarriere in hydrophilen physiologischen Medien besteht.
Ein anderes bekanntes Vehikel ist aus der DE- PS 3815 473 und den parallelen Schutzrechten FR 2631236, US -A- 5776470, US-A 6077520 bekannt. Dieses ist dadurch gekennzeichnet, dass mindestens eine amphiphile und / oder lipophile Komponente mit mindestens einem wasserlöslichen Transferprotein das Wirkstoffsystem bilden.

### Aufgabe vorliegender Erfindung

Es besteht demnach ein Bedarf an speziellen Formulierungen für die topische Anwendung, mittels derer nicht nur ein guter Wirkstofftransport zum Wirkort gesichert wird, sondern womit auch die Effizienz der transportierten Substanzen am Ort verbessert werden kann. Dabei soll insgesamt eine derartige Verbesserung der energetisch / metabolischen Situation erfolgen, dass auch bereits vorhandene Faktoren verstärkt werden können.

### Lösung der Aufgabe

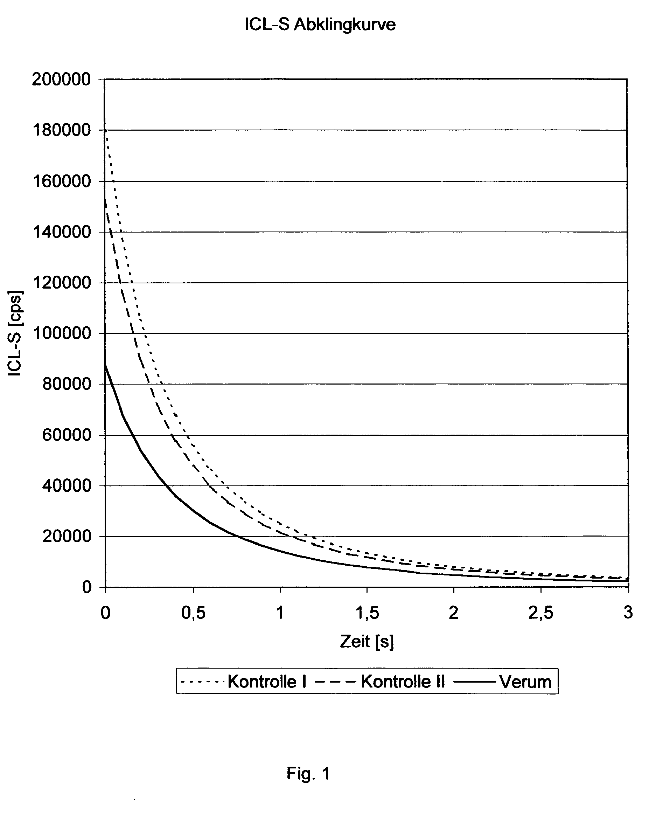

Diese Aufgabe wird erfindungsgemäß dadurch gelöst, dass man topisch applizierbare Zubereitungen bereitstellt, welche eine Kombination aus
a.) einem biokatalytischen Mehrphasensystem M (Microbione), umfassend ein oder mehrere Transferproteine zuammen mit einer oder mehreren lipophilen / amphiphilen Substanzen; und
b.) einem Kapillar- Wirksystem K;
c.) einem geeigneten Lösungsmittel wie Wasser oder Mischungen von Lösungsmitteln mit Wasser,
aufweist. Daneben können für die topische Anwendung geeignete Zusatzstoffe und ggf. Zusatzwirkstoffe vorhanden sein, wobei diese Zusatzwirkstoffe vorzugsweise ausgewählt sind aus einem oder mehreren Haut- und -Anhangsgebilde beeinflussenden Substanzen.
Mit einer derartigen Kombination ist es überraschenderweise möglich, das Angebot an Substraten und Sauerstoff des lokal behandelten Anwendungsfeldes (Haut, Schleimhäute) zu steigern und aufgrund dieser Aktivierung eine verbesserte Effizienz der Wirksubstanzen sowohl der vor Ort transportierten als auch bereits vorhandener oder auf andere Weise zugeführter Stoffe zu erzielen. Damit liegt hier keine herkömmliche, d. h. passive Depotbildung oder Penetrationsförderung vor, sondern eine effizienzsteigernde Überwindung der Energiebarriere zwischen Lipideinheiten im physiologisch hydrophilen Milieu mittels des biokatalytischen Systems sowie eine Erhöhung der Versorgung mit Sauerstoff und Substraten. Es ergibt sich unerwarteter Weise eine überadditive Steigerung der Wirksamkeit gegenüber einem biokatalytischen Mehrphasensystem M (Microbione) einerseits und einem Kapillar- Wirksystem andererseits, wie aus den beigefügten Beispielen und insbesondere Fig. 1 gemäß Beispiel 3 (erfindungsgemäße Zubereitung, Verum) ersichtlich ist.

### Erläuterung der Figur 1

In Figur 1 ist die Wirkung einer erfindungsgemäßen Zubereitung (gemäß Beispiel 3) im ICL-S im Vergleich zu Zubereitungen mit nur einem Kapillarwirksystem bzw. mit nur einem Transfersystem dargestellt. Es ergibt sich in unerwarteter Weise eine überadditive Steigerung.

### Nähere Erläuterung der Erfindung

### Die vorliegende Erfindung betrifft insofern

Topisch applizierbare Zubereitungen, enthaltend eine Kombination aus
a.) einem biokatalytischen Mehrphasensystemen M (Microbione), umfassend ein oder mehrere unterschiedliche Transferproteine zusammen mit einer oder mehreren lipophilen / amphiphilen Substanzen; und
b.) einem Kapillar-Wirksystem K, umfassend ein oder mehrere Wirkmodule, ausgewählt aus
   b1) einem Kapillar-Aktivatorsystem KA aus einem oder mehreren Komponenten, ausgewählt aus Blutfluss steigernden Substanzen; Gefäßwand stabilisierenden Substanzen; Gefäß erweiternden Substanzen;
      und / oder
   b2) einem Kapillar-Protektorsystem KP, aus einem oder mehreren Komponenten, ausgewählt aus Substanzen zur Endothelstabilisierung; Substanzen zum Lipoproteinschutz; Substanzen zur Leukozyten- /Thrombozytenstabilisierung;
      und / oder
   b3) einem Kapillar- Energieversorgungssystem KE aus einem oder mehreren Komponenten, ausgewählt aus Substanzen von Redoxregulatoren bei der Energiebereitstellung; Substanzen als Cofaktoren bei der Energiebereitstellung; Substanzen als Energieträgern;
c) ein Lösungsmittel, ausgewählt aus Wasser, Mischungen von Lösungsmitteln mit Wasser, insbesondere mit Wasser mischbaren Lösungsmitteln;
d) sowie gewünschtenfalls für die topische Anwendung geeignete Zusatzstoffe und / oder Zusatzwirkstoffe.

Sofern vorhanden, können Zusatzwirkstoffe vorzugsweise ausgewählt sein aus einem oder mehreren Haut- und -Anhangsgebilde (Haare und Nägel) beeinflussenden Substanzen.
Insbesondere bevorzugt sind Zubereitungen, worin insgesamt 0,1 bis 85%, bevorzugt 1 bis 60%, insbesondere 1 bis 45%, vor allem 0,1 bis 30 % biokatalytisches Mehrphasensystem M (Microbione); 0,1 bis 30 Gew.%, insbesondere 0,1 bis 25%, bevorzugt 0,2 bis 15% des Kapillar- Wirksystems K und 0,01 bis 40%, bevorzugt 0,1 bis 40%, vor allem 0,1 bis 30% und insbesondere 1 bis 25 % Zusatzstoffe sowie ggf. 0,01 bis 15% , vorzugsweise 0,01 bis 10 Gew.% Zusatzwirkstoffe und als Rest Lösungsmittel, insbesondere Wasser vorhanden sind.

Insbesondere bevorzugt sind Zubereitungen, enthaltend 0,1 bis 30 Gew.% biokatalytisches Mehrphasensystem M; 0,2 bis 15 Gew.% Kapillar- Wirksystem K; 0,01 bis 40 Gew.% Zusatzstoffe (und als Rest Lösungsmittel, insbesondere Wasser). Vor allem ist es hier bevorzugt, wenn weiterhin 0,01 bis 15 Gew.% Zusatzwirkstoffe, welche vor allem ausgewählt sind aus Haut- und - Anhangsgebilde (Haare und Nägel) beeinflussenden Substanzen, enthalten sind. Es ist weiterhin von Vorteil, wenn die Zusatzstoffe hier ausgewählt sind aus Emulgatoren, Co- Emulgatoren, Konditioniermitteln, Konservierungsmitteln, Farbstoffen, Parfümstoffen, Pflegestoffen, Konsistenzgebern, oder Mischungen hiervon.
Als Lösungsmittel (und den Rest der Zubereitungen bildend) ist insbesondere Wasser oder Wasser im Gemisch mit ein- oder mehrwertigen Alkoholen, wie Ethanol, Propanol, Isopropanol, Polyethylenglykol, Polypropylenglykol, ggf. mit Wasser mischbaren z. B. nicht ionischen, amphoteren, anionischen oberflächenaktiven Stoffen, vorhanden.
Ganz besonders bevorzugt sind derartige Zubereitungen in Form von O/W oder W/O Emulsionen. Dann ist als Zusatzstoff wenigstens ein oder mehrere Emulgatoren/ Co- Emulgatoren vorhanden.
Alle Mengen- und Prozentangaben beziehen sich auf das Gewicht der Zubereitungen, sofern nicht anders angegeben.
Besonders geeignet sind weiterhin topische Zubereitungen in Form einer Emulsion (Creme, Lotion), welche ein biokatalytisches Mehrphasensystemen M (Microbione) aus 1 - 45%, ganz besonders 1 bis 18% lipophilen / amphiphilen Substanzen und 0,2 bis 5 % Transferprotein(en); sowie 0,1 bis 10% Kapillar- Wirksystem K; 0,1 - 15% Emulgator(en) (O/W; W/O; emulgierende Kolloide oder Mischungen hiervon und / oder mit Co- Emulgatoren); 0,01 bis 30 Gew.% %, insbesondere 0,1 - 20%, vor allem 0,1 bis 15% Zusatzstoffe; 0,01 - 10 Gew.% Zusatzwirkstoffe und als Rest Wasser aufweisen.

In einer weiteren Ausgestaltung vorliegender Erfindung werden Emulsionen wie vorstehend genannt eingesetzt, welche als Emulgator(en) ein oder mehrere Hydrokolloide, insbesondere solche, ausgewählt aus natürlichen Hydrokolloiden, umfassend Caseinate, Gelatine, Albumine, tierische Proteine (Proteinemulgatoren) mit einer mittleren Molekülmasse von 10 000 D bis etwa 40 000 D; marinen Kolloiden und Derivaten hiervon; oder synthetischen oder partialsynthetisch hergestellten Kolloiden oder Mischungen hiervon, insbesondere Acrylatpolymere (Carbomere) mit einer mittleren Molmasse von mehr als 40 000 D etwa 1 bis 4 Mio D oder Cellulosepolymere mit einer mittleren Molmasse von mehr als 40 000 D, ggf. auch Co- Emulgatoren, aufweisen.
In einer weiteren bevorzugten Ausgestaltung werden die Transferproteine der Microbione ausgewählt aus Lipidtransferproteinen aus tierischen, pflanzlichen, mikrobiellen und gentechnischen Proteinen oder Mischungen hiervon. Besonders geeignet sind Transferproteine aus Pflanzen und tierischen Quellen, vor allem Getreide, Milch.
Insbesondere geeignet ist / sind Lipidtransferprotein(e), das/die ausgewählt ist /sind aus Proteinen aus Getreide mit einer mittleren Molekülmasse von 4 000 bis 10 000 D, Knollenfrüchten oder Früchten mit einer mittleren Molekülmasse von 5 000 bis 20 000 D, tierischen Proteinen, insbesondere aus Milch mit einer mittleren Molekülmasse von 2 000 bis 8 000 D. Proteine aus Seide, Mikroorganismen, marinen Quellen weisen vorteilhafter Weise eine mittleren Molekülmasse von 10 000 bis 40 000 D auf.
Insbesondere geeignet als Transferproteine sind pflanzliche, vor allem solche aus Getreide, vor allem mit einem Molekulargewicht von 4 000 bis 10 000 D, sowie solche tierischen Ursprungs, vor allem Milch, mit einer mittleren Molmasse von 2 000 bis 8 000 D oder auch Gemische hiervon. Bei Gemischen beträgt das Verhältnis von pflanzlichem bzw. Getreideprotein, zu tierischem bzw. Milchprotein bevorzugt 1:1 bis größer 1:1 wie bis 10:1, insbesondere 1,2 bis 5:1.
Als lipophile / amphiphile Komponente eignet(n) sich insbesondere lipophile und / oder amphiphile Komponente(n) ausgewählt aus Kohlenwasserstoffen, natürlichen oder synthetischen Fetten, Ölen, Wachsen, Fettalkoholen, Fettsäureestern, Fettsäurepartialestern, Glyceriden (wie Mono-, Di- Triglyceride), Silikonölen, Silikonwachsen, Lecithinen, Cholesterinen, Phospholipiden, Sphingolipiden, Gangliosiden, Cerebrosiden, Ceramiden, lipophilen oder amphiphilen Pflanzenstoffen, oder Mischungen hiervon.
In einer weiteren bevorzugten Ausgestaltung der Erfindung ist als lipophile /amphiphile Komponente wenigstens eine Substanz aus der Gruppe, umfassend stärker polare Lipide / Amphiphile in Kombination mit wenigstens einer Substanz aus der Gruppe der schwächer polaren Lipide / Amphiphilen ausgewählt, wie sie nachfolgend noch näher beschrieben sind. Zu letzteren gehören besonders bevorzugt Kohlenwasserstoffe, natürliche oder synthetische Fette, Öle, Wachse, Fettsäureester, Fettalkoholether, Polysiloxanverbindungen, Triglyceride. Hiermit werden aus der ersten Gruppe vor allem eine oder mehrere Substanzen aus der Gruppe, umfassend Lecithine, Sphingomyeline, Cholesterine, Phospholipide, Sphingolipide, Ganglioside, Cerebroside, lipophile oder amphiphile Pflanzenstoffen, Mono- /Diglyceride von C₁₂₋₂₂- Fettsäuren, C₁₂₋₂₂Fettalkohole, C₁₂₋₂₂-Fettsäurepartialester von C₂₋₆-Alkoholen, Polyol - C₁₂₋₂₂₋Fettsäureester, kombiniert.
Für das Kapillar-Wirksystem K wird insbesondere ein Kapillar-Aktivatorsystem KA oder ein Kapillar-Aktivatorsystem KA und ein Kapillar-Protektorsystem KP oder ein Kapillar-Energieversorgungssystem KE gewählt; oder auch ein Kapillar-Aktivatorsystem KA, ein Kapillar-Protektorsystem KP und ein Kapillar-Energieversorgungssystem KE.
Ganz besonders bevorzugte Substanzen für das System KA sind Aescin, Roßkastanien- Extrakt, Ginkgo- Extrakt, Arnikablüten- Extrakt, Schafgarben- Extrakt, Steinkleekraut- Extrakt, Mäusedornwurzel- Extrakt, Schachtelhalmkraut-Extrakt oder Kombinationen hiervon; für das System KP Anthocyane, Grüntee-Extrakte, Ω-3- / Ω-6-Fettsäuren oder Kombinationen hiervon; und für das System KE Kreatinphosphat, Arginin und dessen Salze wie Argininphosphat, L-Argininhydrochlorid, NADH, FADH₂, ATP, GTP, Cytochrome, Eisen-Schwefel-Zentren, Coffein, L- Carnithin oder Kombinationen und Derivate hiervon.
Bevorzugt beträgt die Menge an System KA, KP, KE jeweils 0,01 bis 5 %, bezogen auf das Gesamtgewicht der Zubereitung. Insbesondere bevorzugt ist die Gesamtmenge von 0,1 bis 15%, vor allem 0,5 bis 10%.

Ganz besonders bevorzugt sind weiterhin Zubereitungen der beschriebenen Art, welche ein oder mehrere Substanzen, ausgewählt aus Gingko - Extrakt, Mäusedornwurzel- Extrakt, Schachtelhalmkraut- Extrakt, Anthocyanen, Arginin und dessen Salzen, Kreatinphosphat, und ggf. auch Niacin, Folsäure oder Mischungen der einzelnen Substanzen aufweisen.
Bevorzugt sind auch Zubereitungen, welche ein oder mehrere Komponenten gemäß oben genanntem KA und eine oder mehrere Komponenten gemäß oben genanntem KP und / oder ein oder mehrere Komponenten gemäß oben genantem KE aufweisen.
Insbesondere bevorzugt sind Zubereitungen, worin das Kapillar Wirksystem K eine oder mehrere Substanzen, ausgewählt aus Gingko - Extrakt, Arginin und dessen Salzen, Arnikablüten- Extrakt, aufweist.
Als Zusatzstoffe eignen sich galenisch übliche Zusatzstoffe wie, sofern vorhanden Emulgatoren / Co- Emulgatoren wie beschrieben, oder auch andere, insbesondere Konditioniermittel, Konservierungsmittel, Farbstoffe, Parfümstoffe, Pflegestoffe. Diese können vor allem in Mengen von jeweils 0,01 bis 10%, bevorzugt 0,01 bis 5%, vorliegen. Insgesamt beträgt die Menge an Zusatzstoffen bevorzugt 0,01 bis 30, vor allem bis 25 Gew.%.
Als Zusatzwirkstoffe werden insbesondere solche für die Haut- und -Anhangsgebilde (Haare und Nägel) geeigneten eingesetzt. Hierzu gehören z. B. Retinol, Retinolacetat / -palmitat (Vitamin A), Thiaminnitrat, Biotin, Ca-Pantothenat, Pyridoxinsalze, Folsäure, Cyanocobalamin, Riboflavin, Vitamin E, Co- Enzym Q10, Niacin, Magnesiumverbindungen wie -glukonat, -sulfat oder -aspartat, Ursolsäure, Kieselerde, Kieselsäure, Zinkgluconat, pflanzliche Extrakte wie Algen, Papaya, Kamille, Aloe Vera, Aminosäuren wie Cystein, Methionin, Gammalinolensäure (GLA), Linolsäure; Vitamin C (Ascorbinsäure, Calcium-ascorbat / Natrium-ascorbat, Kalium-ascorbat).
Insbesondere bevorzugt sind als Zusatzwirkstoffe Vitamin A, C, E, Co- Enzym Q10, Biotin, Folsäure, Pyridoxinsalze, Niacin, Magnesiumsalze oder Mischungen hiervon.
Vorteilhaft ist es insbesondere, wenn im System auch Niacin (Nicotinsäurederivate), ein oder mehrere B- Vitamine aus B2, B6, B9, B12, insbesondere Niacin und Folsäure, oder Mischungen enthalten sind.

Daneben sind auch andere geeignete Zusatzwirkstoffe wie nachfolgend beschrieben möglich, welche auch dermatologisch / pharmazeutische Eigenschaften aufweisen können.
Die Zusatzwirkstoffe liegen in den jeweils dafür bekannten Mengen, insbesondere 0,01 bis 10, vor allem bis 5 Gew. % bezogen auf das Gesamtgewicht, vor.

### Nähere Beschreibung der einzelnen Systeme

### I. Biokatalytisches Mehrphasensystem M (Microbione)

Die Microbione stellen ein biokatalytisches Mehrphasensystem M dar, welches dynamisch ist und im wesentlichen katalytisch wirksame, insbesondere wasserlösliche, zumindest wasserdispergierbare, Transferprotein(e) sowie die lipoide(n) Substanz(en) (lipophile und / oder amphiphile Stoffe) umfasst. Letztere sind in die lipophilen Taschen der Proteine befüllt, welche weiterhin eine hydrophile Außenstruktur aufweisen. Sie ermöglichen so den Eintrag der lipophilen / amphiphilen Substanz(en), was im Falle von Lipiden zu einer Erneuerung der Membranen führt (und im Falle von lipophilen Zusatzwirkstoffen, die nicht zur Membranstruktur gehören, zu einem verbesserten Transfer). Der dynamische Transfer in die Membranstrukturen der Haut sorgt insofern für einen raschen strukturellen Aufbau der Membranen.
Geeignete Lipidtransferproteine werden bevorzugt ausgewählt aus tierischen, pflanzlichen und mikrobiellen Proteinen. Sie können auch synthetisch oder auch gentechnologisch gewonnen werden.
Diese Proteine weisen daher insgesamt eine mittlere Molekülmasse zwischen 2 000 und 40 000 D, insbesondere 2 000 bis 30 000 D, vor allem 2 000 bis 15 000 D auf.
Insbesondere geeignet als Transferproteine sind solche aus Getreide mit einer mittleren Molekülmasse von 4 000 bis 10 000 D, Proteine aus Knollenfrüchten oder Früchten mit einer mittleren Molekülmasse von 5000 bis 20 000 D, Proteine aus Milch mit einer mittleren Molekülmasse von 2 000 bis 8 000 D, Proteine aus Seide, Mikroorganismen, gentechnischen Quellen mit einer mittleren Molekülmasse von 10 000D, vor allem 15 000 D bis 40 000 D, oder Mischungen hiervon.
Als Knollenfrüchte eignen sich Kartoffeln, weitere geeignete Früchte sind Ylang Ylang, Topinambur oder ähnliche. Marine Quellen sind beispielsweise Algen, Muscheln, Meeresfrüchte. Geeignete Mirkoorganismen sind Hefe, Bakterien.
Besonders bevorzugt sind als Lipidtransferproteine solche aus Getreide, vor allem jene, die aus Weizen, Gerste, Mais und vor allem auch Hafer erhältlich sind.
Ferner eignen sich vor allem auch tierische Proteine aus Milch sowie entsprechende Hydrolysate (mittlere Molekülmasse, vor allem 2 000 bis 4 000 D).
In einer weiteren bevorzugten Ausführungsform der Erfindung werden Proteine aus Getreide wie insbesondere Hafer und aus tierischen Quellen, wie insbesondere Milch, kombiniert.
Seidenproteine werden bevorzugt aus Seidenraupenprotein und Spinnseidenprotein ausgewählt.
Ganz besonders bevorzugt werden Proteine der genannten Art mit einer mittleren Molekülmasse von 2 000 bis 30 000 D, insbesondere 2 000 bis 15 000 D gewählt. Hierunter sind ganz besonders Pflanzenproteine (Getreideproteine) geeignet.
In einer weiteren Ausführungsform sind besonders Milchproteine und auch Seidenproteine mit dem angegebenen Molekulargewichtsbereich geeignet.
Besonders geeignet sind auch Mischungen von Lipidtransferproteinen wie z. B. Mischungen aus pflanzlichen Proteinen und tierischen Proteinen, oder aus marinen Produkten mit pflanzlichen und / oder tierischen Proteinen.
Die Proteine können auf bekannte Weise durch Dispergieren in Wasser, Zentrifugation und Chromatographie erhalten werden, wobei weitere dem Fachmann bekannte Aufarbeitungsmethoden wie Homogenisierung, Entfettung mit organischen Lösungsmitteln, Ausfällen, Dialyse, Filtration zur Anwendung kommen können, vgl. die vorgenannte US-A- 5,776,470. Weitere Methoden zum Erhalt von Proteinen dieser Art sind z. B. in der EP- A 0 620 979 und darin genannter Literatur beschrieben.
Auf diese Weise werden Proteine bzw. Hydrolysate hiervon mit den genannten Molekülmassen gewonnen, die dann in den erfindungsgemäßen Zubereitungen eingesetzt werden. Sie weisen wie erwähnt eine insbesondere katalytische Transferaktivität auf, welche nach dem bekannten Resonanz - Energie - Transfer Test (RET- Test) bestimmt werden kann, vgl. Nichols, J.W., Pagano R.E., J. Biol. Chem. 258 (1983), 5368-5371.
Werden Kombinationen eingesetzt, insbesondere aus tierischen Proteinen und pflanzlichen Proteinen der genannten Art, kann dies z. B. im Verhältnis 1:1 bis 1:10, insbesondere 1:1,2 bis 1:5 erfolgen. Vor allem geeignet ist Milchprotein, insbesondere hydrolysiertes Milchprotein in Kombination mit Pflanzenprotein (z. B. Haferprotein) oder gentechnischen Proteinen im jeweils angegebenen Molekülgewichtsbereich.
Die Transferproteine liegen bevorzugt in einer Menge von 0,1 bis 10 Gew.% vor.

Das biokatalytische Mehrphasensystem M (Microbione) umfasst wie erwähnt weiterhin geeignete lipophile / amphiphile Stoffe, welche wie erläutert eingelagert und dann transferiert werden. Hierzu gehören lipophile oder amphiphile Komponenten, und zwar sowohl stärker polare Substanzen als auch schwächer polare Substanzen.
So können vor allem aus der Gruppe der schwächer polaren Substanzen natürliche oder synthetische (gesättigte, ungesättigte) Fette /Öle bzw. Wachse eingesetzt werden. Hierzu gehören natürliche, partialsynthetische oder synthetische Öle, Kohlenwasserstoffe wie Squalene, insbesondere auch flüssige Paraffine, Isoparaffine, Dioctylcyclohexane, Isodecan, Isohexadecane.
Auch Fettsäureester, z.B. C₂₋₁₈-Alkoholfettsäureester wie Isopropylfettsäureester (Palmitat, Myristat, Isostearat, Oleat), Decyl Oleate, Hexyl Laurate, C 12 - 15 Alkyl Benzoate , Dicaprylyl Carbonate sowie verzweigte Fettsäureester wie Cetearyl Octanoate, Di-n-Butyladipate sind geeignet;
Weiterhin sind C₈₋₂₂- Fettalkoholether wie Dicaprylyl Ether oder Fettalkoholester wie C₁₂₋₁₃- Alkyl Lactate, insbesondere Glyceride (C₁₂₋₂₂ Fettsäure-Glyceride) wie Triglyceride, insbesondere mittelkettige (Neutralöle) wie Caprylic/Capric Triglyceride sowie deren Polyolester wie Propylene Glycol Dicaprylate/ Dicaprate geeignet.

Auch Natürliche (gesättigte/ungesättigte) Fette und Öle finden Anwendung wie Sonnenblumen-, Soja-, Pfirsichkern-, Aprikosenkern-, Ricinus-, Erdnuß-, Mandel-, Nerz-, Weizenkeim-, Sesam- Distel- Mandel-, Avocadoöl, Shea Butter oder Illipé Butter; Natürliche flüssige Wachse, z.B. Jojobaöl oder dessen Substitut Oleyl Erucate;
Weiterhin geeignet sind auch Silikonderivate wie Silikonöle und -wachse, z.B. Polydimethylsiloxane wie Dimethicone, Cyclomethylsiloxane wie Cyclopentasiloxane, Phenylmethylpolysiloxane wie Phenyl Dimethicone oder Alkyl-Polymethylsiloxan-Copolymere wie Cetyl Dimethicone, Stearyl Dimethicone Dialkoxydimethylpolysiloxane wie Stearoxy Dimethicone, Behenoxy Dimethicone;
Geeignet sind auch Wachse wie natürliches oder gebleichtes Bienenwachs (C₂₀₋₄₀- Alkylstearat) oder Micro Wax oder Kohlenwasserstoffwachse wie Mineral Wax sowie hydriertes Rizinusöl oder synthetische Wachse wie Cetylpalmitat oder Myristylmyristat, oder Stearylstearate.
Als stärker polare Lipid - Komponenten sind C₈₋₂₂-Fettalkohole wie - Oleylalkohol, Octyldodecanol, Cetyl/stearylalkohol; geeignet;
Auch C₁₂₋₂₂ - Fettsäurepartialester von mehrwertigen C₂₋₆- Alkoholen wie insbesondere Monoglyceride, oder Diglyceride z. B. Glycerolmono / Distearat, Mischungen hiervon; Polyol C₁₂₋₂₂ - Fettsäureester wie PEG-7- Glyceryl Cocoate, Propylen Glykol Dicaprylate /dicaprate, Gemische wie Hexyldecanol und Hexyldecyl Laurat können eingesetzt werden.
Besonders geeignet sind hier auch Lecithine wie Soja- Eilecithin; Phospholipide, wie Phosphatidylcholin, Phosphatidylserin, Phosphatidylethanolamin, Phosphatidylglycerol, Phosphatidylinositol, Ganglioside, Cerebroside, Cholesterin und Ceramide, wie z.B. Ceramid-2, -3, -6 sowie Sphingolipide.
Ganz besonders bevorzugt werden die Lipidkomponente(n) ausgewählt aus den genannten Kohlenwasserstoffen, (C₈₋₂₂-) Fettalkoholen, -ethern, (C₂₋₆Alkohol)- (C₁₂₋₂₂) Fettsäurepartialester, (Polyol- C₁₂₋₂₂-) / C₂₋₁₈- Fettsäureester, Mono- Di/ Tri (C₁₂₋₂₂-) Glyceriden, natürlichen oder synthetischen Ölen oder Fetten, Lecithinen, Phospolipiden, Ceramiden, Sphingolipiden, Gangliosiden, Cerebrosiden, Cholesterinen, Silikonölen, Silikonwachsen, lipophilen oder amphiphilen Pflanzenstoffen oder Mischungen hiervon.

Insbesondere sind flüssige Paraffine, natürliche Fette/Öle/Wachse, Mono/Triglyceride von C₁₂₋₂₂- Fettsäuren, C₁₂₋₂₂Fettalkohole, C₁₂₋₂₂₋Fettsäurepartialester von C₂₋₆-mehrwertigen Alkoholen , Polyol C₁₂₋₂₂- Fettsäureester, Lecithine, Phospholipide, hierunter vor allem die oben erwähnten, Ganglioside, Cerebroside, Cholesterin, Ceramide, Sphingolipide, Polysiloxanverbindungen, lipophile oder amphiphile Vitamine und Mischungen hiervon geeignet.
Es hat sich ferner gezeigt, dass eine besonders vorteilhafte Ausgestaltung erfindungsgemäßer Zubereitungen dann erhalten wird, wenn die lipophilen /amphiphilen Substanzen eine Kombination aus einer oder mehreren stärker polaren Substanzen und einer oder mehreren schwächer polaren Substanzen darstellen. Zu den schwächer lipophilen Substanzen zählen Kohlenwasserstoffe, natürliche oder synthetische Fette oder Öle oder Wachse, Triglyceride, Silikonderivate wie beschrieben, insbesondere Polysiloxanverbindungen. Zu den stärker polaren Substanzen gehören Lecithine, Ceramide, Cholesterine, Phospholipide, Sphingolipide, Ganglioside, Cerebroside, lipophile oder amphiphile Pflanzenstoffe, Mono- /Diglyceride von C₁₂₋₂₂- Fettsäuren, C₁₂₋₂₂₋Fettalkohole, C₁₂₋₂₂-Fettsäurepartialester von mehrwertigen C₂₋₆- Alkoholen, C₂₋₆- Polyol- C₁₂₋₂₂- Fettsäureester.
In einer weiteren bevorzugten Ausführungsform werden diese Lipide bzw. Lipid-Kombinationen mit den Transferproteinen mit einer mittleren molaren Masse von 2 000 bis 30 000 D (gemäß der jeweiligen Gruppe) zusammen eingesetzt, insbesondere solchen, ausgewählt aus tierischen oder pflanzlichen oder tierischen und pflanzlichen Transferproteinen, im jeweils angegebenen Molekülmassenbereich. Ganz besonders bevorzugt sind als Lipidkomponenten Ceramide, Cholesterole, Mono- Di- /Triglyceride von C₁₂₋₂₂-Fettsäuren, C₁₂₋₂₂₋Fettalkohole und Fettsäureester /Partialester wie beschrieben, Kohlenwasserstoffe oder Mischungen hiervon.
Ganz besonders bevorzugt können auch Silikonderivate wie beschrieben, insbesondere Polysiloxane, vor allem in Verbindung mit o.g. bevorzugten Lipidkomponenten bzw. Kombinationen hiervon eingesetzt werden.
Die lipophile(n) / amphiphile(n) Komponente(n) liegen in Mengen von 0,1 bis 60 Gew.%, bevorzugt 1 bis 45, und ganz besonders 1 bis 25 Gew.% vor.

### II. Kapillar-Wirksystem K

Das Kapillar-Wirksystem K weist ein oder mehrere, also wenigstens ein System, ausgewählt aus KA, KP, KE wie beschrieben auf.
Die Zubereitungen können also ein Kapillar-Aktivatorsystem KA oder ein Kapillar- Protektorsystem KP oder ein Kapillar-Energieversorgungssystem KE oder Kombinationen aus 2 oder 3 Vertretern dieser Gruppe aufweisen. Insbesondere ist ein Kapillar-Aktivatorsystem KA enthalten.
Vorzugsweise umfasst das Kapillar-Wirksystem K ein System KA zusammen mit einem System KP. In einer weiteren bevorzugten Ausführungsform liegt eine Kombination aus KA, KP und KE oder aus KA und KE vor.
Dabei hat sich gezeigt, dass das Kapillar -Aktivatorsystem KA aufgrund blutzirkulatorischer Eigenschaften eine Aktivierung dieses Systems bedingt. Das System KA ist somit aktivierend und kann selbst überadditive, bis gegebenenfalls synergistische Effekte bei Kombination von Substanzen aus jeweils zwei oder drei der genannten Gruppen (Blutfluß steigernd, Gefäßwand schützend, Gefäßwand stabilisierend) auslösen.
Das System KP führt insbesondere zu einer Endothelstabilisierung, einem Lipoproteinschutz sowie einer Leukozyten- / Thrombozytenstabilisierung, wodurch zusätzlich Schadwirkungen z. B. durch Oxidantien zurückgedrängt und damit die Regeneration des Endothels erleichtert wird.
Das System KE dient der energetischen Stabilisierung.
Insbesondere kann durch Kombination aus aktivierendem System KA, dem protektiven System KP oder mit dem energetisierenden System KE oder auch Kombinationen hiervon, z. B. KE +KP, KA + KE, oder alle drei, zusammen mit dem biokatalytischen Mehrphasensystem M (Microbione) auch ein überadditiver bis gegebenenfalls synergistischer Effekt erzielt werden. Dieser lässt sich anhand geeigneter Methoden (wie ICL-S- Messung, oder andere) nachweisen. Die besondere Wirksamkeit einer erfindungsgemäßen Zubereitung gegenüber Vergleichszusammensetzungen ist anhand eines nachfolgenden Anwendungsbeispiels und in Fig. 1 mittels ICL- S-Messung aufgezeigt.
Die für die einzelnen Systeme KA, KP, KE einzusetzenden Komponenten mit den genannten Eigenschaften wie angegeben sind dem Fachmann an sich bekannt. Beispielhaft können die folgenden, insbesondere auch bevorzugten Substanzen genannt werden:
Kapillar - Aktivatorsystem KA:
   Weinblätter-Extrakt, Rutin, Aescin, Roßkastaniensamen-Extrakt, Ginkgo- Extrakt, Arnikablüten- Extrakt, Schafgarben- Extrakt, Steinkleekraut- Extrakt, Mäusedornwurzel- Extrakt, Arnikablüten- Extrakt, Schachtelhalmkraut- Extrakt oder Kombinationen hiervon.
Kapillar -Protektorsystem KP:
   Anthocyane, Grüntee- Extrakte; α- Liponsäure, Ω3- / Ω-6 Fettsäuren (z.B. Borretschöl, Leinöl, Traubenkernöl, Johannisbeersamenöl, Nachtkerzenöl, Lachsöl, Schwarzkümmelöl).
Kapillar- Energieversorgungssystem KE:
   Kreatinphosphat, Arginin und dessen Salze wie Argininphosphat, L- Argininhydrochlorid, NADH, FADH₂, ATP, GTP, Cytochrome, Eisen-Schwefel-Zentren oder Kombinationen hiervon.
   Besonders bevorzugte Ausführungsformen sind dadurch gekennzeichnet, dass sie ein Kapillar-Aktivatorsystem KA und ein Kapillar-Protektorsystem KP / oder ein Kapillar-Energieversorgungssystem KE (Kombination KA, KE); oder auch solche, die neben dem Kapillar-Aktivatorsystem KA ein Kapillar- Protektorsystem KP und ein Kapillar- Energieversorgungssystem KE aufweisen.
   Ganz besonders bevorzugte Substanzen für das System KA sind Aescin, Ginkgo- Extrakt, Arnikablüten- Extrakt, Mäusedornwurzel- Extrakt oder Kombinationen hiervon; für das System KP Anthocyane, α - Liponsäure; und für das System KE Arginin und dessen Salze wie - phosphat, L- Hydrochlorid, Kreatinphosphat oder Kombinationen hiervon.
   Bevorzugt beträgt die Menge an System KA, KP, KE jeweils 0,01 bis 15 Gew.%, bezogen auf das Gesamtgewicht der Zubereitungen. Insbesondere bevorzugt sind Mengen von 0,01 bis 10%, vor allem 0,01 bis 6%.
   Alle Mengen- und Prozentangaben beziehen sich auf das Gewicht der Zubereitungen, sofern nicht anders angegeben.
   Ganz besonders bevorzugt sind weiterhin Zubereitungen der beschriebenen Art, worin das Kapillar- Aktivatorsystem KA ein oder mehrere Substanzen, ausgewählt aus Gingko- Extrakt, Mäusedornwurzel- Extrakt, Roßkastanien- Extrakt; das Kapillar- Protektorsystem KP ein oder mehrere Substanzen, ausgewählt aus Grüntee- Extrakten, bzw. das Kapillar- Energieversorgungssystem KE ein oder mehrere Substanzen, ausgewählt aus Arginin und dessen Salzen, Kreatinphosphat, Eisen- Schwefel- Zentren oder Mischungen derartiger Substanzen aus KA /KE/KP oder deren Kombinationen aufweist. Insbesondere bevorzugt sind Zusammensetzungen, worin das Kapillar Wirksystem K Gingko - Extrakt, Kreatinphosphat, Arginin oder dessen Salze oder Mischungen hiervon aufweist.
In erfindungsgemäßen Zubereitungen, vor allem der oben beschriebenen Art, ist es besonders vorteilhaft, Zusatzwirkstoffe, ausgewählt aus Retinol, Retinolacetat / -palmitat (Vitamin A), Thiaminnitrat, Biotin, Ca-Pantothenat, Pyridoxinsalze, Folsäure, Cyanocobalamin, Niacin, Vitamin C (Ascorbinsäure, Calcium-ascorbat / Natrium-ascorbat, Kalium-ascorbat), Vitamin E (z. B. Tocopherylacetat), Co- Enzym Q10, Magnesiumverbindungen wie Glukonate, -sulfat, Ursolsäure, Kieselerde, Kieselsäure, Zinkverbindungen, pflanzliche Extrakte wie solche aus Algen, Papaya, Kamille, Aloe vera, Aminosäuren wie Cystein, Methionin, Gammalinolensäure (GLA), Linolsäure, einzusetzen.
Insbesondere bevorzugt sind als Zusatzwirkstoffe Vitamin E, Co- Enzym Q10, Biotin, Folsäure, Niacin, Pyridoxin bzw. dessen Derivate, Magnesiumsalze, oder Mischungen hiervon.
Die einzelnen Substanzen und deren einzusetzenden bzw. bevorzugt in topischen Produkten einsetzbaren Mengen sind dem Fachmann bekannt.
Eine weitere geeignete Ausführungsform sieht KA oder KP oder die oben beschriebene Kombination aus KA und KP wie beschrieben zusammen mit Wirkstoffen wie Niacin (Nicotinsäure, deren Derivaten, insbesondere Nicotinsäureamid, oder Kombinationen hiervon) vor.
Die einzelnen Substanzen aus den genannten Gruppen sind für sich bekannt bzw. können nach dem Fachmann bekannten Verfahren hergestellt werden.

### III. Zusatz-/ Zusatzwirkstoffe

Die erfindungsgemäßen Zubereitungen können wie erläutert, Zusatzstoffe in einer Menge von 0, insbesondere 0,1 bis 40%, insbesondere 1 bis 35 Gew.%, bevorzugt 5 bis 30 Gew. %, vor allem 5 bis 25 Gew. % aufweisen.
Üblicherweise umfassen topische Verabreichungsformen Wasser und ggf. weitere geeignete Lösungsmittel. So können Anwendungsformen wie gelartige Produkte, z. B. mit Wasser, ggf. enthaltend ein- oder mehrwertige Alkohole wie Ethanol, (Iso)propanol, Glycerin, Polyethylen/- propylenglycol, oder Konditioniermittel, oder ein Tonic z. B. in Form einer Lösung in Wasser / Alkohol oder anderen geeigneten Mittel, z. B. oberflächenaktive Stoffe oder Mischungen hiervon, erhalten werden, wobei die jeweils gewünschte Konzentration eingestellt werden kann. Es können auch Dispersionen und Emulsionen erhalten werden.
Besonders bevorzugte topische Darreichungsformen sind Cremes / Lotionen /Fluids z. B. auf O/W- oder W/O- Basis. Hierzu wird die erfindungsgemäße Zusammensetzung in Form einer Emulsion erhalten.
Zu den bevorzugten Zusatzstoffen gehören sofern vorhanden Emulgatoren /Co- Emulgatoren, Konditioniermittel, Konservierungsmittel, Farbstoffe, Parfümstoffe, Pflegestoffe, Zusatzwirkstoffe oder Mischungen hiervon. Diese können insbesondere in Mengen von jeweils 0,01 bis 20%, bevorzugt 0,1 bis 15%, vorliegen.
Zu üblichen Konditioniermitteln in geeigneten Mengen gehören beispielsweise Acrylamide (Polyacrylamid, oder Polyacrylamidhaltige Gemische), Stärke, wie beispielsweise Hypromellose oder Stärkeglycola, Reis-, Weizen-, Mais- und Kartoffelstärke, ferner auch hydrophobisch modifizierte Stärken (Corn Starch Modified), Aluminium Starch Octenylsuccinate, Hydroxypropyl Starch Phosphate Ester oder dessen Gemische wie Aluminium Starch Octenylsuccinate, Acrylates Copolymer, Magnesium Carbonate, und ähnliches. Femer sind hier auch Polysaccharide (z. B. Xanthan Gum), oder Silikate wie Magnesium Aluminium Silikat zu nennen.
Als Konditioniermittel können weiterhin auch Komplexbildner wie EDTA Na-Salz, Mittel zur pH- Einstellung, z.B. Zitronensäure, Natronlauge, Lösungsmittel wie Propylenglykol oder Alkohole, Salze wie NaCl, Stärke, bzw. Stärke-Derivate oder Mischungen hiervon enthalten sein. Weitere Konditioniermittel sind insbesondere Mono- und Dialkylphosphate oder Glycerylstearat.
Besonders geeignete Pflegestoffe sind Phytosterole (im wesentlichen Gemische aus ß-Sitosterol, Campesterol und Stigmasterol oder ethoxilierte Derivate hiervon) wie solche aus Rapsöl. Auch Phosphatidylcholin, Phosphatidylserin oderdiethanolamin, oder Mischungen hiervon mit den oben genannten Phytosterolen sind hier verwendbar. Weiterhin können hier Silikonöle z.B. Dimethicone oder Silikon -(Co)Polymere, z.B. Di- Cyclomethicon oder Divinyldimethicone/Dimethicone Copolymer, C12-13 Pareth-3/C12-13 Pareth-23) Verwendung finden. Weitere Pflegestoffe sind Feuchthaltemittel wie Glycerin, Propylenglykol oder Polyethylenglykole, Polypropylenglykol, Butylenglykol, Sorbitol oder Polymere, z.B. Polyquaternium-Typen wie Polyquaternium -39, Collagen oder dessen Hydrolysate, Aminosäuren, Harnstoff, Polysaccharide (Biosaccharide Gum-1), Glucosaminoglykane, z.B. Hyaluronsäure oder sulfatierte Glucosaminoglykane wie Chondroitinsulfat, Dermatansulfat, Keratansulfat, Heparansulfat, insbesondere deren Na-Salze oder Heparin-Na, Glukane, z.B. β-Glukan, z.B. Hafer β-Glukan, Mannane wie Konjac Mannane, oder handelsübliche Feuchthaltemittel wie Inositol, Salze, z.B. Natriumlactat, DL-2-Pyrrolidon-5-Carbonsäure, Na-Salz. Bevorzugt sind Polyethylen-/ Propylenglykol oder sowie Polysaccharid-Verbindungen, Algenextrakte und/oder Hyaluronsäure, Na-Salz.
Weitere mögliche Zusatzstoffe sind geeignete, insbesondere wasserlösliche oberflächenaktive Stoffe wie nicht ionische polyoxyethylierte Substanzen, Sarcosinate, Sulfate, Acylglutamate, Isothionate, Polyglycoside u. ä.
Daneben können gegebenenfalls ein oder mehrere Konservierungsmittel vorhanden sein. Geeignete Konservierungsmittel sind beispielsweise lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutarononitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, Iso-Butylparaben, bevorzugt Methyl- oder Propylparaben oder Mischungen hiervon oder Climbazol oder geeignete Kombinationen der genannten Stoffe wie z.B. Methyl-, Propylparaben und Sorbinsäure. Insbesondere geeignet sind auch Mischungen.
Geeignete Parfümstoffe (z.B. bevorzugt 0,01 bis 5%) sind z.B. die unter Zusatzwirkstoffen genannten etherischen Öle oder handelsübliche Parfümmischungen.
Als Farbstoffe (z.B. bevorzugt 0,01 bis 2%) werden vorzugsweise die für gattungsgemäße Produkte bekannten Komponenten gewählt.
Im Falle von Emulsionen werden je nach Ziel (O/W bzw. W/O) entsprechende Emulgatoren eingesetzt.
Geeignete Zusatzvvirkstoffe sind die vorstehend genannten und bevorzugten Haut- und - Anhangsgebilde (Haare und Nägel) beeinflussenden Zusatzwirkstoffe, weiterhin auch UV- Filter bekannter Art (wie UVA, UVB Filter, z. B. Zimtsäureester, 4-Methylbenzyliden Camphor, Paraaminobenzoesäure und -ester, Benzophenone-3; Methyl Anthranilate, Analoga, Titandioxid u-ä.; Pflanzenextrakte, etherische Öle, dermatologisch/ pharmazeutische Wirkstoffe wie insbesondere antimikrobiell wirksame Substanzen (Antibiotika, Antimykotika, antivirale Stoffe), Hormone oder hormonwirksame Stoffe wie Sexualhormone, Wundreinigungsmittel, Analgetika, Cytokine, Zytostatika, Antiaknemittel, Immunsuppressiva, Pigmentierungsregulatoren, Mittel gegen Photodermatosen oder vasoaktive / antiphlogistische Substanzen oder Kombinationen hiervon; adstringierende und sebumregulierende Substanzen wie Hydroxydecanoic Acid, Caproyl Collagen Aminoacids, Sebacic Acid, Cinnamonum zeylanicum.
Es können auch kühlende/beruhigende Substanzen als Zusatzwirkstoffe wie Menthyl Lactate oder Sodium Hyaluronate, Wheat Germ Extrakt, Saccharomyces Cerevisiae Extrakt inkorporiert werden.
Darüber hinaus sind als Zusatzwirkstoffe weiterhin auch Alpha- und Betahydroxysäuren und deren Derivate, z. B. Glykol-, Äpfel-, Zitronen-, Wein-, Milchsäure, Salicylsäure, Isopropylbenzylsalicylate, C12 - 13 Alkyl Lactate Minoxidil oder auch antiphlogistische und antibakterielle Substanzen wie Triterpene, z. B. Ursolsäure, Glycyrrhizinsäure oder Glycyrrhetinsäure und deren Derivate, z. B. Stearyl Glycyrrhetinate, Kaliumglycyrrhinat; Pantothensäurederivate, z. B. Panthenyltriacetat; Allantoin; Bisabolol; Azulene, z. B. Charri-Azulene oder Guaj-Azulen; Phytosphingosine; Triclosan; Chlorhexidin-Derivate einsetzbar.

Weiterhin können sekundäre Pflanzenstoffe (Polyphenole, Ferulasäure und deren Ester oder Isoflavone wie Soja-Isoflavone oder Hyaluronsäure, Oleanolsäure) als wirksame Zusatzstoffe eingesetzt werden.
Besonders bevorzugte Zusatzwirkstoffe sind die vorstehend als Haut - und Anhangsgebilde (Haare und Nägel) beeinflussende Substanzen bezeichneten Mittel, oder Kombinationen hiervon mit Zusatzstoffen wie UV Filtern, Farbstoffen und / Parfümstoffen, Konservierungsmitteln.
Bei wasserhaltigen Formulierungen in Form von Emulsionen können Cremes oder Lotionen erhalten werden. Es können auch gelartige Formulierungen erhalten werden, indem man das oder die Transferproteine in Wasser zusammen mit dort löslichen Zusatz- bzw. ggf. Zusatzwirkstoffen vorlegt, sodann die lipophile / amphiphile Substanz ggf. unter Erwärmung und Hinzufügen eines-weiteren Lösungsmittels wie genannte Alkohole, z.B. Ethanol oder sowie weiterer Zusatzstoffe, sofern vorhanden, in an sich bekannter Weise einarbeitet.
Zur Herstellung von Emulsionen werden geeignete Emulgatoren eingesetzt.
Als O/W-Emulgatoren, welche vorteilhafter Weise einen HLB- Wert von 9-18, bevorzugt 9-15 und insbesondere 9-13, aufweisen, eignen sich insbesondere nichtionische polyoxyethylierte/polyoxypropylierte Produkte wie PEG/PPG- Ester, Ether von C8-C22 Fettsäuren, -Fettalkoholen mit Ethoxylierungs/ Propoxylierungsgraden von 2 bis 150, insbesondere 5 bis 40, z. B. Polyethylenglycole mit geeignetem Ethoxylierungsgrad; wie z.B, wie z.B. Polyoxyethylenglykol-400―monostearat, oleylether, -monolaurat, -sorbitanmonolaurat, - sorbitantristearat. Weiterhin geeignet sind nichtionische und ionische Phosphate, ionische einwertige Salze, (Poly)glycerylester, Zuckerester, Sterol-Derivate, Rizinusöl-Derivate, Siloxane hiervon mit dem jeweils geeigneten HLB- Wert größer 8 oder Gemische hiervon. Es können auch Mischungen, insbesondere auch mit Co-Emulgatoren eingesetzt werden.
Die Menge an diesen Emulgatoren /Co- Emulgatoren beträgt gewöhnlich 0,1 bis 15 Gew.%, bevorzugt 1 bis 10, insbesondere 1 bis 5 Gew. %
Darüber hinaus sind hier auch andere topisch verträgliche Substanzen zur Emulgierung möglich, welche dem Fachmann hierfür bekannt sind.
Der W/O-Emulgator weist vorteilhafter Weise einen HLB- Wert von 1-8, insbesondere 1-7, bevorzugt 2-7 und ganz besonders bevorzugt 3-6 auf.
Als W/O-Emulgatoren sind insbesondere bevorzugt Sorbitan- Derivate, z. B. Sorbitan Ester wie Sorbitan Oleate, Sorbitan Stearate, Sorbitan Sesquioleate , Sorbitan Isostearate, Sorbitan Tristearate; Polyethoxylierte Produkte wie Polyethoxylierte Fettsäuren und -alkohole/ ester wie PEG-2 Oleate (HLB = 5,0), PEG-4 -Distearate (HLB = 3,0), PEG-2 Stearate (HLB = 4,4), Ceteareth-3, (HLB = 5,0), Ceteth-2 (HLB = 5,3); Ethoxylierte Triglyceride wie PEG-5 Castor Oil (HLB = 3,9), PEG-6 Diricinoleate (HLB = 5,0), PEG-7 Hydrogenated Castor Oil (HLB = 5,0); Cetylstearylalkohol, Glycerinmonostearat, Fettsäuresalze (Poly)glycerylderivate wie Polyglycerylester wie Polyglyceryl-3 Diisostearate (HLB = 3,5), Polyglyceryl-2 Dipolyhydroxystearate (HLB = 3,5), Diisostearoyl Polyglyceryl-3 Diisostearate (HLB ca. 5), Polyglyceryl-3 Oleate (HLB ca. 5), Polyglyceryl-3 Dioleate (HLB ca. 5), Polyglyceryl-4 Isostearate (HLB ca. 5); Glycerin-Ester wie Glyceryl Ricinoleate (2,7), Glyceryl Laurate (HLB = 4,9), Glyceryl Dioleate S/E (HLB = 2,9);Polyolester wie Glycol Oleate (HLB = 2,7), Glycol Ricinoleate (HLB = 2,0), Glycol Dilaurate (HLB = 2,0), Propylene Glycol Ricinoleate (HLB = 3,6), Propylenglykol Laurate (HLB = 2,7);Glucose-Derivate: Glucose Ester wie Methyl Glucose Dioleate (HLB ca. 5), Methyl Glucose Isostearate (HLB ca. 5); Pentaerythrit-Derivate wie Pentaerythrit-Fettsäureester, z.B. Pentraerythrityl Mono-laurate (HLB = 4,8), Pentaerythrityl Monotallate (HLB = 4,0) oder Mischester, z.B. mit Citronensäure Fettalkoholester wie Dicocoyl Pentaerythrityl Distearyl Citrate, Sorbitan Sesquioleate, Cera Alba, Aluminium Stearate, HLB = 4,0), Dicocoyl Pentaery-thrityl Distearyl Citrate, Cera Microcristallina, Glyceryl Oleate, Aluminium Stearate, Propylene Glycol, HLB = 4,0);Polymere wie Polyoxypropylen-polyoxyethylen-Blockpolymere (INCI-Name: Poloxamere), z.B. Pluronic® PE 3100 (HLB = 4,5), Pluronic® PE 6100 (HLB = 3,0) oder PEG-30 Dipolyhydroxystearate (Arlacel® P 135, HLB ca. 5,5); Siloxan-Derivate wie Polysiloxan-Copolymere, Polysiloxan-Polyether-Copolymere, insbesondere Polysiloxan-Polyalkyl-Polyether-Copolymere wie Cetyl Dimethicone Copolyol (Abil® EM 90, HLB ca. 5), Laurylmethicone Copoyol (Dow Corning® Q2-5200, HLB ca. 4); Siloxan-Derivate-haltige Emulgatorgemische wie Abil® EM 90, Arlacel ®582 und Magnesiumstearat oder Mischungen hiervon oder das handelsübliche Abil® WE 09 (HLB ca. 5) bestehend aus Cetyl Dimethicone Copolyol, Polyglyceryl-4 Isostearate und Hexyl Laurate.
Bevorzugt sind ethoxylierte Produkte wie Fettsäuren und Triglyceride wie PEG-2 Oleate, PEG-7 Hydrogenated Castor Oil (Cremophor® WO 7), (Poly)glycerylester sowie Glucose -Ester, und Polysiloxan-Copolymere oder Mischungen hiervon.
Die Mengen betragen hier etwa 1 bis 20, vorzugsweise bis 15 Gew.%.
Es können wie erwähnt auch Co- Emulgatoren vorhanden sein, wie z.B. Cetyl/Stearylalkohol, Glycerinmonostearat.
Als Emulgatoren eignen sich gemäß einer bevorzugten Ausführungsform auch Hydro(Schutz) kolloide. Insbesondere geeignet sind insofern aus der Gruppe der natürlichen Vertreter (tierische) Caseinate, Proteinderivate tierischer Natur, Gelatine, Albumine, oder Mischungen hiervon. Als Proteinderivate tierischer Natur (oder entsprechend synthetisch hergestellte Produkte) eignen sich Hydrolysate mit einer mittleren Molekülmasse von mehr als 10 000 D .Ganz besonders bevorzugt sind als Kolloide wasserlösliche Caseinverbindungen, insbesondere Salze von Casein, wie Natriumcaseinat.
Als Gelatine eignen sich die Typen A und B gemäß Ph.Eur. 4. Ebenfalls geeignet sind Albumine aus Ei, Serum, die chromatographisch aus dem natürlichen Ausgangsmaterial gewonnen werden können.
Die genannten Verbindungen sind an sich bekannt.
Zu den synthetisch / partial synthetischen Kolloiden gehören bevorzugt wasserlösliche (Poly)(Meth)Acrylatpolymere wie Carboxy-Vinyl-Mischpolymerisate mit hohem Molekulargewicht (1 - 3 Mio.) sowie deren (neutralisierte oder zu neutralisierenden ) Copolymere. Es können auch Acrylat-Copolymere eingesetzt werden, z.B. Arylates/C10-30 Alkyl Acrylate Crosspolymer, oder Acrylamidalkylsulfonsäurederivate wie Ammonium Acryloyldimethyltaurate/ VP Copolymer, vgl. Ph.Eur.4 . Weiterhin möglich sind hier wasserlösliche Cellulosederivate wie Methylcellulose, Hydroxypropylcellulose, Hydroxyproylcellulose, Hydroxypropylmethylcellulose. Diese letztgenannten Kolloide weisen insbesondere eine mittlere molare Masse von mehr als 40 000 D , z. B. Carbomere 1,25 bis 4 Mio. auf.
Das oder die Kolloide werden bevorzugt in Mengen von 0,01 bis 10 Gew.%, vor allem 0,01 bis 5 Gew.% eingesetzt, und vor allem 0,1 bis 4 Gew.%. und haben sowohl eine Schutzfunktion als auch eine emulgierende Wirkung, derart, dass bei derer Einsatz auf herkömmliche Emulgatoren, insbesondere ONV Emulgatoren, verzichtet werden kann und die Transferproteine weiterhin ihre katalytische Funktion ausüben.
Je nach Gesamtzusammensetzung der Zubereitung erhält man mit diesen Kolloiden auch O/W und W/O Emulsionen.

Die Art und Menge der Zusatzstoffe/ Zusatzwirkstoffe richtet sich nach der gewünschten Anwendungsform und dem gewünschten Zweck. Es ist bevorzugt, wenn höchstens 30%, bevorzugt 0,01 bis 25%, vor allem auch 0,1 bis 25% oder 1 bis 20 %, insbesondere 3 bis 20% an Zusatzstoffen und 0,01 bis 15, insbesondere 0,01 bis 10, vor allem 0,01 bis 7 Gew %. Zusatzwirkstoffe vorhanden sind.
Alle Mengenangaben beziehen sich auf Gewichtsprozent.

Mit den erfindungsgemäßen Zubereitungen werden somit 2 Systeme in besonders wirksamer Weise miteinander verknüpft, nämlich das biokatalytische Mehrphasensystem M (Microbione) und das Kapillar- Wirksystem K, mit Kapillaraktivator KA, und / oder Kapillarprotektor KP und / oder Kapillar-Energieversorgungssystem KE. Mit einer derartigen Kombination wird überraschender Weise ein überaddtitiver Effekt erzielt, wie im nachfolgenden Anwendungsbeispiel erläutert. Das Zusammenwirken beider Systeme M + K ist in nachfolgendem Schema dargestellt:

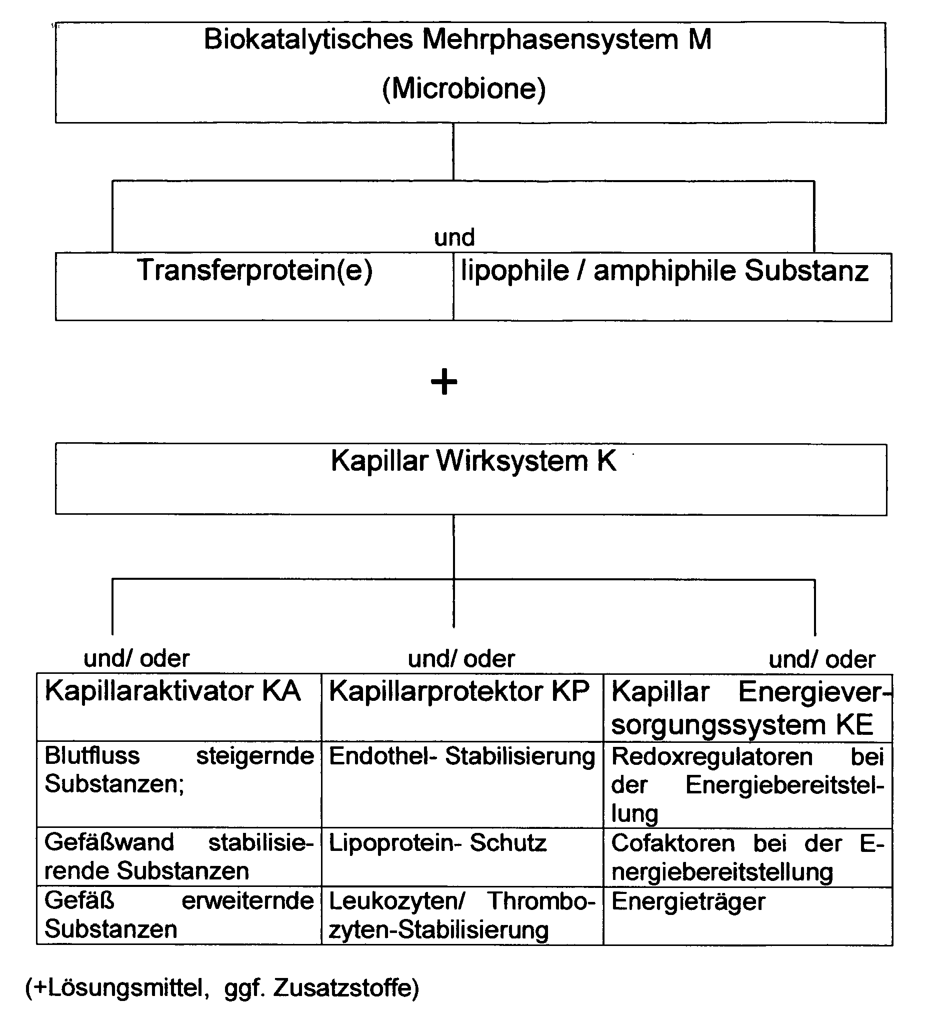

In einer besonders geeigneten Ausführungsform der Erfindung liegen die Zubereitungen in Form einer Emulsion vor, worin das oder die Transferproteine, ausgewählt aus Getreide mit einer mittleren Molekülmasse von 4000 bis 10 000 D, Proteine aus Knollenfrüchten oder Früchten mit einer mittleren Molekülmasse von 5000 bis 20 000 D, Proteine aus Milch mit einer mittleren Molekülmasse von 2 000 bis 8 000 D, Proteine aus Seide, Mikroorganismen, gentechnischen Quellen mit einer mittleren Molekülmasse von 10 000 D, vor allem 15 000D bis 40 000 D, oder Mischungen hiervon, insbesondere aus Getreide- und / oder Milchproteinen (mittlere Molekülmasse 4 000 bis 10 000 D bzw. 2 000 bis 8 000 D), z. B. 0,5 bis 4 Gew.%; zusammen mit den genannten lipophilen / amphiphilen Substanzen, insbesondere Lecithinen, Sphingolipiden, Cholesterinen, Phospholipiden, Gangliosiden, Cerebrosiden, Ceramiden, lipophilen oder amphiphilen Pflanzenstoffen, (Mono- Di- Tri-)Glyceriden, Kohlenwasserstoffen, natürlichen oder synthetischen Fetten, Ölen, Wachsen, Fettalkoholen, Fettsäureestern, Fettsäurepartialestern, Silikonölen, Silikonwachsen oder Mischungen hiervon; z. B. 1 bis 45 Gew.%; vorliegt und weiterhin emulgierende Kolloide (z. B. 0,2 bis 7 Gew.%), ausgewählt aus tierischem Protein mit einer mittleren Molekülmasse von mehr als 10 000 D bis 40 000 D, Caseinaten, Carbomeren, Acrylaten, insbesondere den genannten tierischen Proteinen (Proteinemulgatoren), Caseinaten; sowie Zusatzstoffen wie Co- Emul-gatoren, (z. B. 1 bis 5 Gew.%) Konsistenzgebern, Konditioniermitteln, Konservierungsmitteln, Farbstoffen, Parfümstoffen, Pflegestoffen oder Mischungen hiervon sowie Zusatzwirkstoffen, ausgewählt aus UV- Filtern, Haut- und -Anhangsgebilde beeinflussenden Substanzen, wie vor allem Tocopherylacetat, CoEnzym Q10, Magnesiumsalzen, Niacin, Folsäure, oder Mischungen hiervon, in jeweils bekannten Mengen neben System K vorliegen. Als Wirksystem K können ein oder mehrere Substanzen, ausgewählt aus Roßkastanien- Extrakt, Ginkgo- Extrakt, Anthocyanen, Kreatinphosphat, Arginin und dessen Salzen, ATP vorhanden sein.

### Herstellung

Die erfindungsgemäßen Zubereitungen werden hergestellt, indem man im Falle von Emulsionen die lipophilen / amphiphilen Stoffe, die Transferproteine, Wasser und ggf. Zusatz- bzw. Zusatzwirkstoffe sofern deren Temperaturstabilität gegeben ist, bei höheren Temperaturen wie zwischen 20 und 80 °C in Gegenwart eines geeigneten Emulgators emulgiert und nach dem Abkühlen, weitere temperaturlabile Zusätze sowie insbesondere dann das oder die Wirkmodule des Kapillar Wirksystems K hinzufügt. Im Falle von Tonics, Gelen oder Dispersionen wird der Wassergrundlage ein entsprechender Lösungsvermittler bzw. Konsistenzgeber wie Alkohole oder oberflächenaktive Mittel bzw. Konditioniermittel hinzugesetzt und sodann die einzelnen Komponenten wie angegeben inkorporiert (es können herkömmliche hierfür in der Kosmetik/ Dermatologie bekannte Vorrichtungen / Umsetzungsbedingungen dabei eingesetzt werden). Vorteilhafter Weise werden hier als lipophile / amphiphile Substanzen des biokatalytischen Systems M (Microbione) insbesondere stärker polare Komponenten, vor allem in Mengen von 0,1 bis 10%, eingesetzt.

### Anwendung

Die erfindungsgemäßen Zubereitungen eignen sich zur topischen Anwendung, d.h. zur kosmetischen (nicht therapeutischen) und auch zur dermatologischen /therapeutischen Anwendung auf Haut, Haar, Nägel oder Schleimhäute bei Säugern, insbesondere Menschen. Flüssige Zubereitungen wie Tonics, Spray oder auch Gele (z. B. zur Dusche) können daher aufgesprüht oder eingerieben werden. Emulsionen (wie Creme, Lotion, Milch) oder Masken werden durch Einreiben auf die Haut verteilt. Insbesondere geeignet sind die Zubereitungen zur vor allem kosmetischen Anwendung, oder auch zur dermatologischen Anwendung/ Behandlung /Vorbeugung, bei funktionsgestörter Haut, wie vor allem trockener, empfindlicher, lichtgeschädigter, insbesondere bei alternder Haut, oder auch bei Akne, als UV- Schutz mit Aufbaufunktion, oder auch zur Behandlung entzündlicher Prozesse wie Psoriasis, Akne, Verbrennungen bei Säugern, insbesondere Menschen. Die Zubereitung kann dabei mehrmals täglich angewendet werden. Besonders bevorzugt ist die Anwendung in Form einer Creme (Tages- Nacht- Nähr- Aufbau, Schutzcreme, Sonnencreme etc.), insbesondere bei kosmetischer Anwendung.
Mittels der erfindungsgemäßen Kombination können die eingesetzten Transferproteine noch effektiver ihre Wirkung als Vehikel für lipophile / amphiphile Komponenten entfalten, insbesondere zur, vor allem biokatalytischen, Regeneration von Membranstrukturen und zur Erneuerung der zellulären und extrazellulären Lipidstrukturen, vor allem der Haut (z. B. Altershaut). Durch das Kapillar- Wirksystem erfolgt zusätzlich eine Erhöhung der Versorgung mit Sauerstoff und Substraten, wobei unerwarteter Weise ein überadditiver Effekt erzielt wird, wie in nachfolgendem Beispiel 6 gezeigt. So kann z. B. neben der allgemeinen kosmetischen Anwendung für Pflege, oder dem Einsatz bei funktionsgestörter Haut, insbesondere der Altershaut, wie beschrieben, auch ein therapeutischer Einsatz z. B. bei entzündlichen Zuständen, Reizzuständen der Haut z. B. infolge Allergie, oder Hautverbrennungen, bei Reizzuständen von Schleimhäuten wie z. B. der Atemwege z. B. infolge Allergie, oder Irritationen vorgenommen werden. Die Art der Anwendung (insbesondere kosmetisch, oder auch z. B. dermatologisch) kann insofern z. B. auch von der Art des oder der Zusatzwirkstoffs/ e abhängen.

### Beispiele

Die Erfindung wird anhand der nachfolgenden Beispiele näher erläutert (Mengenangaben in Gewichtsprozent):

### Erfindungsgemäße Zubereitungen

### Beispiel 1: O/W Creme mit Kapillar Wirksystem aus KA +KP +KE

### Biokatalytisches Mehrphasensystem M (Microbione):

| | |
|---|---|
| Avena Sativa (Haferprotein) | 0,6% |
| C12-15 Alkyl Benzoate | 5,00% |
| Squalane | 4,00% |
| Cetearyl Isononanoate | 3,00% |
| Kapillar Wirksystem K: | |
| Gingko Extrakt (KA) | 1,00% |
| Grüntee- Extrakt (KP) | 1,00% |
| Kreatinphosphat (KE) | 0,5% |
| Zusatzstoffe: | |
| Q 10 | 0,50% |
| Tocopheryl Acetate | 1,00% |
| Cetyl Alcohol | 2,00% |
| Hydroxypropylethylcellulose | 0,50% |
| Konservierungsmittel | q.s. |
| Aqua | ad 100 |

Die ONV Creme mit den beschriebenen Inhaltsstoffen wurde hergestellt, indem man Wasser + Transferprotein sowie Fettkomponenten zusammen mit Cetylalkohol und Hydroxypropylmethylcellulose bei 30 bis 60° C in an sich bekannter Weise (in geeigneten Apparaturen) emulgiert und sodann die restlichen Zusatz(wirk)stoffe sowie die Wirkmodule aus Kapillar Wirksystem K bei reduzierten Temperaturen einarbeiten.

### Beispiel 2. W/O Creme

### Analog wurde folgende Zubereitung hergestellt:

### Biokatalytisches Mehrphasensystem M (Microbione):

| | |
|---|---|
| Hydrolysiertes Milchprotein | 0,3% |
| Avena Sativa (Haferprotein) | 0,5% |
| Decyl Oleate | 5,0% |
| Caprylic/Capric Triglyceride | 15,00% |
| PEG- Hydrogenated Castor Oil | 15,00% |
| Kapillar Wirksystem K: | |
| Aescin (KA) | 1,00% |
| NADH (KE) | 2,00% |
| Zusatzstoffe: | |
| Niacin | 0,50% |
| Glycerin | 1,00% |
| Parfüm | q.s. |
| Konservierungsmittel | q.s. |
| Aqua | ad 100 |

### Beispiel 3 ONV Creme (Verum gemäß Anwendungsbeispiel 6.)

### Analog zu Beispiel 1 wurde folgende Zusammensetzung bereitet:

### Biokatalytisches Mehrphasensystem M (Microbione)

| | |
|---|---|
| Milchprotrein (mittl. MW 5 000 D) | 0,6% |
| Octyldodecanol | 5,00% |
| Caprylic/Capric-Triglycerid | 5,00% |
| Kapillar Wirksystem K: | |
| Roßkastanien-Extrakt (KA) | 0,8% |
| L-Arginin- Hydrochlorid (KE) | 0,2% |
| Zusatzstoffe: | |
| Glycerylstearat | 2,00% |
| Natriumcaseinat | 2,5% |
| Glycerin | 1,0% |
| Konservierungsmittel | q.s. |
| Aqua | ad 100 |

### Kontroll- (Vergleichs-)Zubereitungen

### Beispiel 4 = Kontrolle I analog zu Beispiel 3 ohne biokatalytisches Mehrphasen System M (Microbione)

Analog zu Beispiel 3 wurde folgende Zubereitung hergestellt:

| | |
|---|---|
| Octyldodecanol | 5,00% |
| Caprylic/Capric-Triglycerid | 5,00% |
| Kapillar Wirksystem K: | |
| Rosskastanien- Extrakt (KA) | 0,8% |
| L-Arginin- Hydrochlorid (KE) | 0,2% |
| Zusatzstoffe: | |
| Glycerylstearat | 2,00% |
| Natriumcaseinat | 2,5% |
| Glycerin | 1,0% |
| Konservierungsmittel | q.s. |
| Aqua | ad 100 |

### Beispiel 5 = Kontrolle II analog zu Beispiel 3, ohne Kapillaraktivator

### Analog zu Beispiel 3 wurde folgende Zusammensetzung hergestellt:

### Biokatalytisches Mehrphasensystem M (Microbione):

| | |
|---|---|
| Milchprotein (mittl. MW 5 000 D) | 0,6% |
| Octyldodecanol | 5,00% |
| Caprylic/Capric-Triglycerid | 5,00% |
| Zusatzstoffe: | |
| Glycerylstearat | 2,00% |
| Natriumcaseinat | 2,5% |
| Glycerin | 1,0% |
| Konservierungsmittel | q.s. |
| Aqua | ad 100 |

### 6. Anwendungsbeispiel / Vergleich (Fig.1)

36 freiwillige ausgesuchte hautgesunde Probandinnen wurden in 3 Gruppen aufgeteilt. Je 12 pro Gruppe erhielten über einen Zeitraum von 4 Monaten eine Zubereitung gemäß Beispiel 3, 4 oder 5 direkt appliziert auf die Haut (Rücken), und zwar in einer Menge von 2 mg / cm² (entspricht 120 mg/ Areal), 1 mal täglich.
Die Überlegenheit der Kombination eines biokatalytischen Mehrphasensystems mit einem Kapillar-Wirksystem läßt sich anhand der ICL- S- Messung zeigen. Bei diesem Verfahren wird bei freiwilligen Probanden das betreffende Hautareal mit ultraviolettem Licht bestrahlt und anschließend die Photonenemission aus der bestrahlten Haut mittels "Single Photon Counting" gemessen. Je geringer die Photonenemission, um so besser der Schutz der Haut vor oxidativen Schäden. Bei Anwendung der Zubereitung gemäß vorliegender Erfindung auf der Haut wird hinsichtlich der die Hautstruktur verbessernden Eigenschaften ein Effekt erreicht, der sich UV-Licht- protektiv messbar nachweisen läßt. Diese Tatsache dient als Wirksamkeitsnachweis für die überlegene Formulierung gemäß der erfindungsgemäßen Kombination.

### Methodenbeschreibung:

Zu Beginn und am Ende der Anwendung der jeweiligen Zubereitung wurde auf dem betreffenden Hautareal die sogenannte ICL-S (induced chemiluminescence of human skin) gemessen.
Hierbei wird eine Kuppel aus Flüssigkeits-Lichtwellenleitern mit einem zentral angeordneten Lichtwellenleiter zur Bestrahlung in einem optimierten Abstand und Winkel zur Haut lokalisiert, ohne diese zu berühren. Die Lichtwellenleiter befinden sich in einem Messkopf, der fest auf die Haut gedrückt wird. Diese Anordnung erlaubt einen definierten Photonentransfer von der Haut zum Detektor und kompensiert Einflüsse wie Schwitzen oder Bewegungen der Testperson.
Je Person wurden vier Untersuchungsareale zwei Minuten mit UVA (320 ≤ λ ≤ 400 nm) bestrahlt (Bestrahlungsenergie 10mW/cm², λmax = 350 nm). Unmittelbar nach diesem UV-Stress wurde die Photonenemission der Haut mittels Single-Photon-Counting über einen Zeitraum von 200 s gemessen. Die Menge an gle-Photon-Counting über einen Zeitraum von 200 s gemessen. Die Menge an freigesetzten Photonen korreliert dabei mit dem durch UVA-Strahlung ausgelösten "oxidativen Stress" [Sauermann G, Mei WP, Hoppe U, Stäb F: Ultraweak photon emission in vivo: Influence of topically applied antioxidants on human skin. Methods Enzymol. 199; 300:419-428].
Der Mittelwert der integralen Photonenemission sowie die mittleren Abklingkinetiken der von der Haut emittierten Photonen wurden vor und nach Anwenduung der Zubereitung berechnet und auf signifikante Unterschiede getestet.
Unterschiede im Hautkolorit wurden rechnerisch berücksichtigt.
Im Rahmen der vorliegenden Untersuchung konnte belegt werden, dass die Intervention mit der erfindungsgemäßen Kombination zu einer Stärkung der antioxidativen Schutzfunktionen der Haut führt. Hierbei wurden nicht, wie sonst üblich, oxidierte Metabolite indirekt nachgewiesen, es erfolgte vielmehr eine direkte Messung der UV-induzierten Chemilumineszenz der Haut bei den einzelnen Probandinnen. Diese Verbesserung der Schutzfunktion der Haut ist nicht nur wesentlich im Sinne eines dermalen Anti-Aging-Effektes, sondern auch im Sinne der Prävention z. B. vieler altersabhängiger Erkrankungen der Haut, die mit oxidativen Belastungen zusammenhängen.

## Patentansprüche

1. Topisch applizierbare Zubereitungen, enthaltend eine Kombination aus
a.) einem biokatalytischen Mehrphasensystemen M (Microbione), umfassend ein oder mehrere unterschiedliche Transferproteine zusammen mit einer oder mehreren lipophilen / amphiphilen Substanzen; und
b.) einem Kapillar-Wirksystem K, umfassend ein oder mehrere Wirkmodule, ausgewählt aus
b1) einem Kapillar - Aktivatorsystem KA aus einem oder mehreren Komponenten, ausgewählt aus Blutfluss steigernden Substanzen; Gefäßwand stabilisierenden Substanzen; Gefäß erweiternden Substanzen;
und / oder
b2) einem Kapillar-Protektorsystem KP, aus einem oder mehreren Kom ponenten, ausgewählt aus Substanzen zur Endothelstabilisierung; Substanzen zum Lipoproteinschutz; Substanzen zur Leukozyten- /Thrombozytenstabilisierung;
und / oder
b3) einem Kapillar- Energieversorgungssystem KE aus einem oder mehreren Komponenten, ausgewählt aus Substanzen von Redoxregulatoren bei der Energiebereitstellung; Substanzen als Cofaktoren bei der Energiebereitstellung; Substanzen als Energieträgern;
c) ein Lösungsmittel, ausgewählt aus Wasser, Mischungen von Lösungsmitteln mit Wasser; insbesondere mit Wasser mischbaren Lösungsmitteln;
d) sowie gewünschtenfalls für die topische Anwendung geeignete Zusatzstoffe und / oder Zusatzwirkstoffe.

2. Zubereitungen gemäß Anspruch 1, **dadurch gekennzeichnet, dass** Zusatzstoffe ausgewählt sind aus Emulgatoren / Co-Emulgatoren, Konditioniermitteln, Konservierungsmitteln, Farbstoffen, Parfümstoffen, Pflegestoffen, Konsistenzgebern, und / oder Zusatzwirkstoffe oder Mischungen hiervon enthalten sind.

3. Zubereitungen gemäß einem der Ansprüche 1 oder 2, **dadurch gekennzeichnet, dass** 0,1 bis 30 Gew. % biokatalytisches Mehrphasensystem, 0,2 bis 15 Gew.% Kapillar- Wirksystem K, 0,01 bis 40 Gew. % Zusatzstoffe, ggf. 0,01 bis 15 Gew. % Zusatzwirkstoffe sowie Lösungsmittel, insbesondere Wasser, als Rest enthalten sind.

4. Zubereitungen gemäß Anspruch 3, **dadurch gekennzeichnet, dass** weiterhin 0,01 bis 15 Gew.% Zusatzwirkstoffe enthalten sind.

5. Zubereitungen gemäß Anspruch 3 oder 4, **dadurch gekennzeichnet, dass** die Zusatzstoffe ausgewählt sind aus Emulgatoren / Co-Emulgatoren, Konditioniermitteln, Konservierungsmitteln, Farbstoffen, Parfümstoffen, Pflegestoffen, Konsistenzgebern oder Mischungen hiervon.

6. Zubereitungen gemäß Anspruch 1 bis 5, **dadurch gekennzeichnet, dass** sie ein Kapillar-Aktivatorsystem KA aufweisen.

7. Zubereitungen gemäß Anspruch 1 bis 6, **dadurch gekennzeichnet, dass** sie ein Kapillar-Aktivatorsystem KA und ein Kapillar-Protektorsystem KP und /oder ein Kapillar-Energieversorgungssystem KE aufweisen.

8. Zubereitungen gemäß einem der Ansprüche 1 bis 7, **dadurch gekennzeichnet**, sie in Form einer O/W oder W/O Emulsion vorliegen.

9. Zubereitungen gemäß Anspruch 8, **dadurch gekennzeichnet, dass** sie 1 - 45%, ganz besonders 1 bis 18% lipophile / amphiphile Substanzen, 0,2 bis 5 % Transferprotein(e), 0,1 bis 10% Kapillar-Wirksystem K, 0,1- 15% Emulgator, ausgewählt aus O/W-, W/O Emulgatoren, emulgierenden Kolloiden oder Mischungen hiervon oder mit Co- Emulgatoren, 0,1-20%, insbesondere 0,1-10%, Zusatzstoffe, 0,1- 10% Zusatzwirkstoffe und als Rest Wasser aufweisen.

10. Zubereitungen gemäß einem der Ansprüche 8 oder 9, **dadurch gekennzeichnet, dass** als Emulgator(en) ein oder mehrere Kolloide, ausgewählt aus natürlichen Hydrokolloiden, umfassend Caseinate, Proteinemulgatoren tierischer Natur mit einer mittleren Molekülmasse von mehr als 10 000 D, Gelatine, Albumine; marine Kolloide und Derivate hiervon; oder synthetischen oder partialsynthetisch hergestellten Kolloiden oder Mischungen hiervon oder mit Co- Emulgatoren, enthalten sind.

11. Zubereitungen gemäß Anspruch 1 bis 10, **dadurch gekennzeichnet, dass** das/die Transferprotein(e) ausgewählt sind aus Lipidtransferproteinen aus tierischen, pflanzlichen, mikrobiellen und gentechnischen Proteinen oder Mischungen hiervon.

12. Zubereitungen gemäß Anspruch 11, **dadurch gekennzeichnet, dass** das oder die Transferproteine ausgewählt sind aus pflanzlichen Getreideproteinen mit einer mittleren Molekülmasse von 4 000 bis 10 000 D, und Milchproteinen mit einer mittleren Molekülmasse von 2 000 bis 8 000 D oder Mischungen hiervon.

13. Zubereitungen gemäß Anspruch 12, **dadurch gekennzeichnet, dass** sie eine Mischung aus Getreideproteinen mit einer mittleren Molekülmasse von 4 000 bis 10 000 D, und Milchproteinen mit einer mittleren Molekülmasse von 2 000 bis 8 000 D im Verhältnis 1,2 : 1 bis 5:1 aufweisen.

14. Zubereitungen gemäß Anspruch 1 bis 13, **dadurch gekennzeichnet, dass** die lipoide(n) Substanz(en) ausgewählt ist (sind) aus lipophilen und / oder amphiphilen Komponente(n), ausgewählt aus Lecithinen, Cholesterinen, Phospholipiden, Sphingolipiden, Gangliosiden, Cerebrosiden, Ceramiden, lipophilen oder amphiphilen Pflanzenstoffen, Glyceriden, Kohlenwasserstoffen, natürlichen oder synthetischen Fetten, Ölen, Wachsen, Fettalkoholen, Fettsäureestern, Fettsäurepartialestern, Silikonölen, Silikonwachsen oder Mischungen hiervon.

15. Zubereitungen gemäß Anspruch 1 bis 14, **dadurch gekennzeichnet, dass** 0,01 bis 10 Gew. % Zusatzwirkstoffe, ausgewählt aus Haut- und - Anhangsgebilde (Haare und Nägel) beeinflussenden Substanzen enthalten sind.

16. Zubereitungen gemäß Anspruch 15, **dadurch gekennzeichnet, dass** zusätzlich UV- Filter als Zusatzwirkstoffe enthalten sind.

17. Zubereitungen gemäß Anspruch 15 oder 16, **dadurch gekennzeichnet, dass** Haut- und Anhangsgebilde beeinflussende Substanzen, ausgewählt aus Retinol, Retinolacetat / -palmitat (Vitamin A), Thiaminnitrat, Biotin, Ca-pantothenat (B₅), Pyridoxinsalzen, Folsäure, Niacin, Vitamin E, Co- Enzym Q10, Magnesiumsalzen, Ursolsäure, Kieselerde, Kieselsäure, Zinkverbindungen, pflanzliche Extrakte wie Algen, Papaya, Kamille, Aloe vera, Aminosäuren wie Cystein, Methionin, Gammalinolensäure (GLA), Linolsäure; Vitamin C oder Mischungen hiervon enthalten sind.

18. Zubereitungen gemäß Anspruch 1 bis 17, **dadurch gekennzeichnet, dass** Substanzen, ausgewählt aus Ginkgo- Extrakt, Schachtelhalmkraut- Extrakt, Mäusedornwurzel- Extrakt, Arginin und dessen Salze, Anthocyane, Kreatinphosphat oder Mischungen hiervon sowie Niacin, Folsäure oder Mischungen hiervon enthalten sind.

19. Zubereitungen gemäß Anspruch 1 bis 18, **dadurch gekennzeichnet, dass** sie in Form einer Emulsion vorliegen, enthaltend ein oder mehrere Transferproteine, ausgewählt aus Getreide mit einer mittleren Molekülmasse von 4 000 bis 10 000 D, Proteinen aus Knollenfrüchten oder Früchten mit einer mittleren Molekülmasse von 5 000 bis 20 000 D, Proteinen aus Milch mit einer mittleren Molekülmasse von 2 000 bis 8 000 D, Proteinen aus Seide, Mikroorganismen, gentechnischen Quellen mit einer mittleren Molekülmasse von 10 000 D, vor allem 15 000 D bis 40 000 D, oder Mischungen hiervon, zusammen mit den genannten lipophilen / amphiphilen Substanzen, sowie emulgierenden Kolloiden, ausgewählt aus tierischem Proteinemulgator mit einer mittleren Molekülmasse von mehr als 10000D, Caseinaten, Carbomeren, Acrylaten, insbesondere den genannten Proteinen, Caseinaten, und Zusatzstoffen wie Co- Emulgatoren, Konsistenzgebern, Konditioniermitteln, Konservierungsmitteln, Farbstoffen, Parfümstoffen, Pflegestoffen oder Mischungen hiervon sowie Zusatzwirkstoffen, ausgewählt aus UV- Filtern, Haut- und -Anhangsgebilde beeinflussenden Substanzen und einem Wirksystem K.

20. Zubereitungen gemäß Anspruch 19, **dadurch gekennzeichnet, dass** als Komponenten des Systems K ein oder mehrere Substanzen ausgewählt aus Aescin, Roßkstanienextrakt, Ginkgo- Extrakt, Anthocyanen, Arginin und dessen Salzen, Kreatinphosphat, ATP enthalten sind.

21. Zubereitungen gemäß einem der Ansprüche 1 bis 20, **dadurch gekennzeichnet, dass** sie in Form einer Lotion, einer Creme, einer Milch, eines Gels, eines Sprays, Tonics oder einer Maske vorliegen.

22. Verwendung von Zubereitungen gemäß einem der Ansprüche 1 bis 21 zur kosmetischen Anwendung bei funktionsgestörter Haut / Haaren / Nägeln, bei alternder Haut oder zur Pflege von Haut / Haaren / Nägeln.

23. Verwendung gemäß Anspruch 22 zur Regeneration von Altershaut oder als UV Schutz mit Aufbaufunktion.

24. Verwendung von Zubereitungen gemäß einem der Ansprüche 1 bis 21 zur Herstellung eines Mittels zur dermatologischen Vorbeugung /Anwendung wie z. B. bei Psoriasis, Verbrennungen, bei Reiz- oder allergischen Zuständen der Haut, Schleimhaut.

25. Verwendung von Zubereitungen gemäß einem der Ansprüche 1 bis 21 zur Regeneration von keratinösen, insbesondere Membranstrukturen.
